Europäisches Patentamt

European Patent Office

Office européen des brevets

(19)

(11) Numéro de publication : **0 308 297 B1**

(12) # FASCICULE DE BREVET EUROPEEN

(45) Date de publication du fascicule du brevet :
**14.08.91 Bulletin 91/33**

(51) Int. Cl.⁵ : **A61F 2/32, A61F 2/38**

(21) Numéro de dépôt : **88402240.1**

(22) Date de dépôt : **06.09.88**

(54) Prothèse articulaire totale, notamment de la hanche.

(30) Priorité : **08.09.87 FR 8712428**

(43) Date de publication de la demande :
**22.03.89 Bulletin 89/12**

(45) Mention de la délivrance du brevet :
**14.08.91 Bulletin 91/33**

(84) Etats contractants désignés :
**BE CH DE GB IT LI**

(56) Documents cités :
**EP-A- 0 225 819**
**EP-A- 0 281 984**

(56) Documents cités :
**DE-A- 3 528 151**
**DE-U- 8 623 855**
**FR-A- 2 549 717**
**FR-A- 2 610 824**

(73) Titulaire : **Teinturier, Pierre**
**Champeaux**
**F-63122 Ceyrat (FR)**

(72) Inventeur : **Teinturier, Pierre**
**Champeaux**
**F-63122 Ceyrat (FR)**

(74) Mandataire : **Fort, Jacques**
**CABINET PLASSERAUD 84, rue d'Amsterdam**
**F-75009 Paris (FR)**

## Description

La présente invention concerne les prothèses articulaires totales comprenant deux pièces destinées à être ancrées dans des éléments complémentaires du squelette et présentant des surfaces de glissement ou de roulement relatif permettant de reconstituer aussi exactement que possible les déplacements de l'articulation naturelle.

Elle trouve une application particulièrement importante, bien que non exclusive, dans le domaine des prothèses de la hanche, qui sont à l'heure actuelle les plus implantées et se prêtent particulièrement bien à la mise en oeuvre de l'invention. Elle n'est toutefois pas limitée à ce cas particulier et elle est susceptible d'être également appliquée aux prothèses du genou (qui, comme celles de la hanche, ont à supporter le poids du patient lors de la marche). Une pièce acétabulaire selon les caractéristiques du préambule de la revendication 1 est connue du document EP-A-0225819.

Longtemps on a posé en postulat la nécessité d'une fixation rigide, par scellement, d'au moins un des éléments de la prothèse. Dans le cas par exemple de la prothèse de la hanche, la plupart des prothèses existantes comportent une pièce fémorale massive destinée à être ancrée mécaniquement ou par scellement dans le canal médullaire du fémur et une pièce acétabulaire en forme de cupule destinée à être ajustée de façon serrée dans une cavité ménagée dans le cotyle. Que la cupule soit bloquée par scellement ou par vissage ou qu'elle soit laissée libre de glisser en cas de venue en butée relative des deux pièces lors de leur rotation, la prothèse ne peut s'adapter aux déformations du squelette sous charge.

On a récemment tenté de tenir compte de l'élasticité du fémur en donnant une légère élasticité en flexion à la tige de la pièce fémorale d'une prothèse de la hanche. Une telle constitution n'apporte qu'une solution partielle et incomplète au problème.

A priori on pourrait penser qu'il est exclu de donner à l'ensemble des pièces constitutives d'une prothèse une élasticité leur permettant de s'adapter aux déformations du squelette, car ces déformations tendent à gêner, voire même à bloquer les mouvements relatifs requis pour le jeu de l'articulation. La présente invention vise à fournir une prothèse du type ci-dessus défini répondant mieux que celles antérieurement connues aux exigences de la pratique, permettant d'obtenir l'adaptabilité requise sans incidence défavorable sur les mouvements, grâce à un découplage des fonctions d'adaptation aux déformations du squelette et de maintien d'une géométrie invariable des surfaces de contact.

Dans ce but l'invention propose une prothèse dont les deux pièces présentent des surfaces de frottement ou de roulement relatif ménagées dans des parties indéformables mais dont l'une au moins prend appui sur l'os par l'intermédiaire de moyens élastiquement déformables.

Fréquemment, l'une des pièces de la prothèse est prévue pour avoir une fixation intramédullaire canalisant les contraintes reçues par la prothèse. C'est par exemple le cas de la pièce fémorale dans une prothèse de la hanche. L'absence de mise en charge de la corticale qui en résulte dans des prothèses classiques risque de provoquer une atrophie osseuse. Conformément à un aspect de l'invention, cette atrophie est évitée grâce à des moyens permettant de transmettre les efforts aux corticales. Une première solution consiste à revêtir la partie intramédullaire de la pièce d'un matériau déformable (tel qu'une bague de rattrapage) sur une hauteur choisie en fonction de la nature de la prothèse et de la longueur de la queue intramédullaire. Une autre solution, utilisable éventuellement en liaison avec la première, consiste à prévoir, sous un renflement de la pièce formant sabot et recouvrant la section osseuse, des moyens déformables destinés à appliquer une force axiale de mise en charge des corticales. Une autre solution encore consiste à partir d'une ébauche forgée à un encombrement légèrement supérieur à celui de son logement dans le canal médullaire et à découper, par usinage classique ou électro-érosion, plusieurs portions de la queue sans les détacher de façon à conserver des charnières proximale et distale. On obtient ainsi un appui élastique.

Très souvent également, l'autre pièce d'une prothèse est destinée à être emmanchée dans une cavité taillée dans le cotyle. Dans le cas particulier et particulièrement représentatif d'une prothèse de la hanche, comportant une pièce acétabulaire délimitant un logement en forme de calotte sphérique et une pièce fémorale terminée par un tête en forme de boule, il est particulièrement important que la pièce acétabulaire puisse suivre les déformations sous charge et s'y adapter. Pour cela, la pièce acétabulaire peut avoir diverses constitutions. La pièce peut comporter une cupule rigide interne en un matériau ayant un faible coefficient de frottement et une résistance à l'usure élevée, de nature classique, contenue dans une coquille dont la zone externe présentant une élasticité radiale. La zone externe peut être d'une seule pièce avec la partie interne et constituer un doublage divisé en plusieurs secteurs par des incisions radiales et/ou relié à la partie centrale par une liaison réservée par une saignée circonférentielle. Plusieurs saignées disposées en quinconce dans le sens radial peuvent être prévues pour constituer un doublage multiple augmentant la flexibilité.

Lorsque cette solution est adoptée, on peut introduire la pièce dans une cavité préalablement préparée dont la taille est légèrement inférieure à celle de la coquille implantée, dont le maintien en place sera obtenu grâce à la pression élastique. On peut également prévoir un filetage à filets de forme dissymétrique permettant de visser la pièce, c'est-à-dire conserver un mode de fixation largement utilisé dans les prothèses coxofémorales actuelles.

Une autre solution consiste à donner à la coquille une constitution composite, comprenant un corps métallique rigide et des moyens élastiques rapportés sur le corps pour exercer une pression dans le sens radial et/ou axial. Ce revêtement du corps peut le recouvrir sur une fraction variable de sa surface.

D'autres solutions encore sont possibles : la coquille peut comporter une coque interne rigide et une coque externe déformable délimitant une cavité close remplie d'un matériau amortisseur et capable de répartir les contraintes, par exemple du type liquide très visqueux ou solide pâteux.

L'invention sera mieux comprise à la lecture de la description qui suit de modes particuliers de réalisation, donnés à titre d'exemple non limitatifs. La description se réfère aux dessins qui l'accompagnent, dans lesquels :

— la figure 1 est une vue en élévation et en coupe partielle d'une pièce fémorale de prothèse de la hanche selon un mode particulier de réalisation de l'invention ;

— la figure 2 est une vue de droite de la figure 1 ;

— les figures 3, 4 et 5 sont des vues en coupe suivant les lignes III, IV et V de la figure 1 ;

— les figures 6 et 7, similaires aux figure 1 et 2, montrent une variante de réalisation ;

— la figure 8 est une vue en coupe suivant la ligne VIII-VIII de la figure 6 ;

— les figures 9 et 9A sont des vues de détail montrant respectivement un fragment de bague de tolérance utilisable sur la tige de la pièce fémorale des figures 6 et 7 et l'allure générale d'une bague de tolérance, en vue développée ;

— la figure 10, similaire à une fraction de la figure 1, montre une autre variante ;

— les figures 11 et 12 montrent, respectivement en vue de dessus et en coupe suivant la ligne XII-XII de la figure 11, un mode de réalisation de pièce acétabulaire pour prothèse suivant l'invention ;

— la figure 13, similaire à la figure 12, montre une variante de réalisation de la pièce acétabulaire.

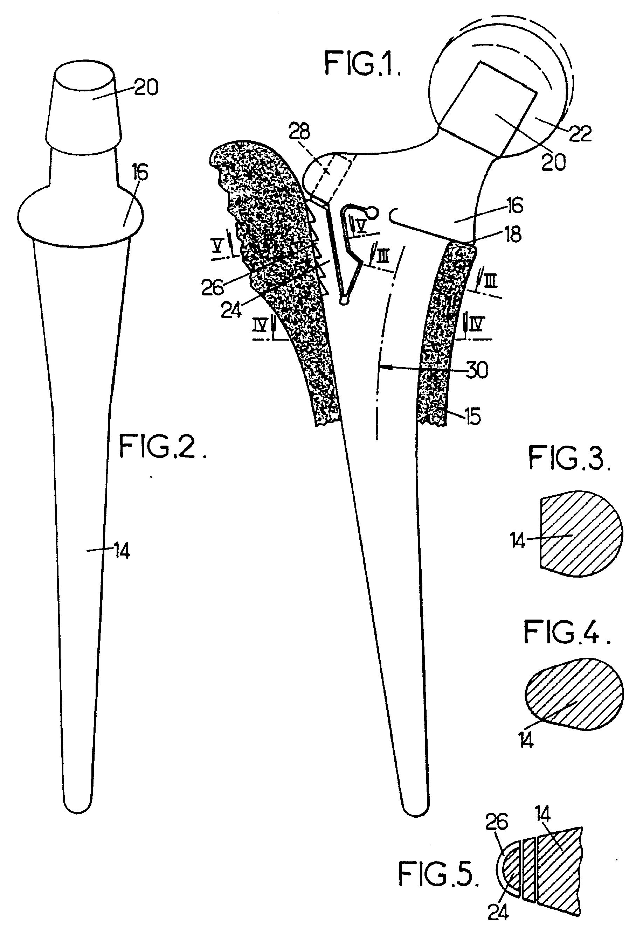

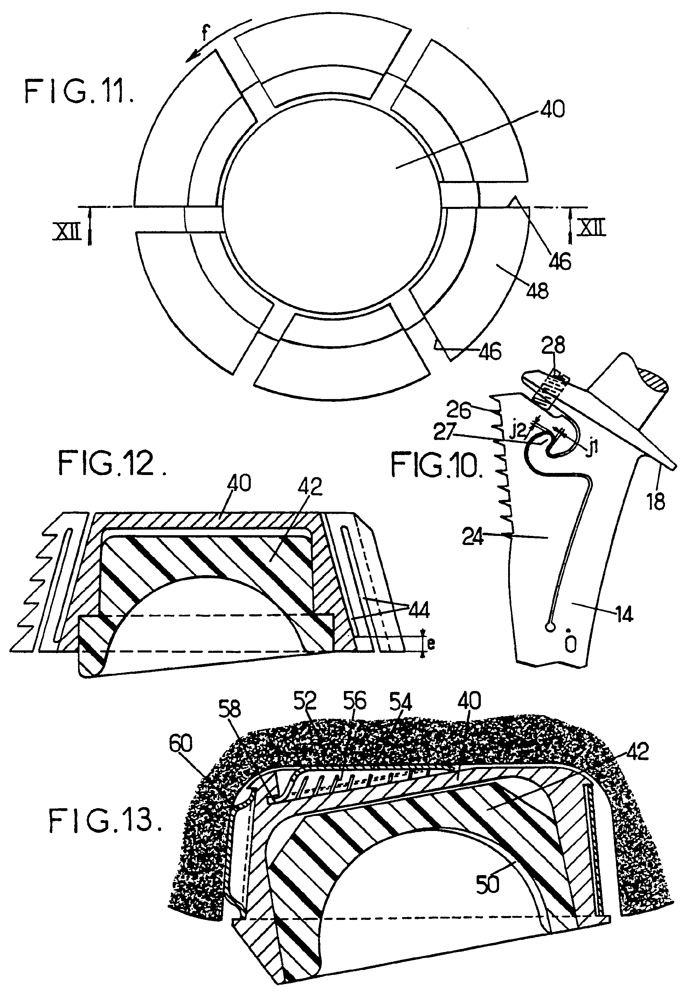

Une prothèse de la hanche suivant l'invention peut comporter une pièce fémorale suivant l'un des modes de réalisation montrés en figure 1 à 10 et une pièce acétabulaire suivant l'un des modes de réalisation montrés en figures 11 à 13, ou des variantes des constitutions décrites.

La pièce fémorale montrée en figures 1 à 6 est constituée par une pièce métallique massive en un métal ou alliage compatible avec les tissus. On peut notamment utiliser les alliages à base de titane (tel que l'alliage UTA 6V), divers aciers inoxydables et l'alliage connu sous le nom de VITALLOY (VITALLOY est une marque de fabrique). La pièce, constituée par usinage d'une ébauche forgée ou matricée, peut être regardée comme ayant une tige 14 d'ancrage dans le canal médullaire du fémur 15, de forme évasée à partir de l'extrémité basse, un renflement 16 présentant un épaulement inférieur 18 d'appui cortical et un cone morse terminal 20 destiné à recevoir une boule 22 (figure 1) en matériau dur à faible coefficient de frottement, en forme de calotte sphérique dont le diamètre est généralement compris entre 22 et 32 mm.

Pour permettre d'adapter la prothèse à l'anatomie des divers patients on prévoit généralement plusieurs boules qui diffèrent les unes des autres par l'emplacement du cone morse et/ou le diamètre de la calotte sphérique, permettant de donner à la surface de frottement différents emplacements par rapport à l'appui cortical comme indiqué par des lignes en tirets sur la figure 1.

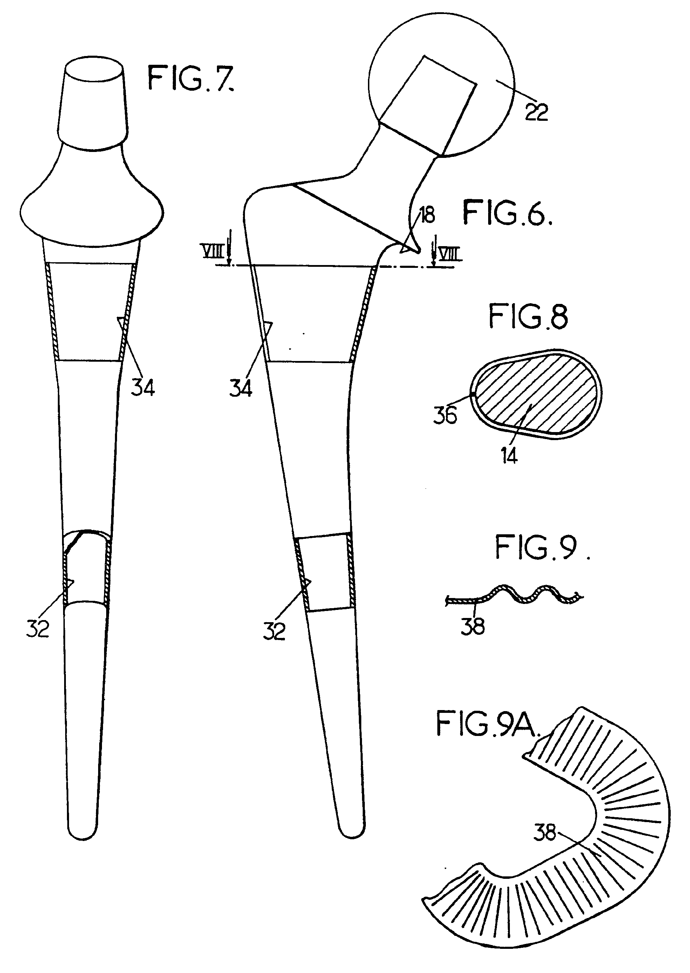

Pour assurer tout à la fois l'ancrage sans scellement de la pièce fémorale et la mise en contrainte axiale permanente de la corticale, la partie haute de la tige fémorale 14 est découpée de façon à constituer un doigt élastique 24 muni d'une crémaillère de retenue 26 (figures 1 et 5). Cette découpe est prolongée de façon à faire apparaître, dans la partie principale de la tige qui se raccorde au renflement 16, une section à contrainte maximale indiquée par la ligne III-III sur la figure 1. De façon à donner à la fente de découpe une épaisseur minimale, la découpe peut être effectuée par électro-érosion à l'aide d'un fil. Un élargissement est prévu à l'extrémité de la fente de découpe et dans l'angle aigu de celle-ci pour éviter l'apparition de contraintes pouvant conduire à une fissuration.

Dans le renflement 16 est ménagé un trou taraudé de réception d'une vis de pression 28 destinée à s'appuyer sur l'extrémité du doigt 24 et à repousser celui-ci dans un sens provoquant son écartement. Cette vis de pression permet à la fois d'ancrer la crémaillère 26 à la paroi médullaire et de mettre la corticale en contrainte axiale. Lors de la mise en place de la pièce fémorale, la vis 28 est desserrée. L'élasticité du doigt 24 tend à le rapprocher de la fibre neutre 30 de la pièce. La pièce fémorale dans cet état est insérée dans le canal médullaire après préparation du fémur et notamment découpe de la surface d'appui de l'épaulement 18. Puis la vis est bloquée.

Dans le mode de réalisation montré en figures 6 à 9A (où les éléments correspondant à ceux des figures 1 à 5 sont désignés par le même numéro de référence), la pièce fémorale est massive et non découpée. Mais elle présente deux gorges tronconiques 32 et 34 de faible profondeur destinées à recevoir des bagues de tolérance (non montrées sur la figure 8). La gorge 32, placée un peu au dessous de la mi-longueur de la tige de la pièce, a une longueur comprise entre 15 et 25 mm (20 mm par exemple). La gorge 34 placée immédiatement au dessous de l'épaulement 18 d'appui sur le fémur à une longueur plus grande, habituellement comprise entre 20 et 30 mm (25 mm par exemple).

Les deux bagues de tolérance ont la même constitution générale. Elles ont pour fonction de maintenir la pièce fémorale dans l'os tout en compensant, les déformations de l'os qui conduisent à une modification de la distance entre l'axe de la tige et la paroi du canal médullaire. Elles peuvent être constituées du même matériau que la pièce fémorale et se présentent sous forme d'une bande pliée dont les extrémités sont séparées par un jeu placé dans le plan médian de la tige côté externe, c'est-à-dire en 36 (figure 8). Chaque bague de tolérance peut avoir une forme ondulée, l'orientation des ondulations étant celle montrée en figure 9A. Une bague 38 de ce genre aura par exemple, lorsqu'elle est prévue pour être placée dans une gorge de 0,8 à 1,2 mm de profondeur, une épaisseur de quelques dixièmes de millimètres avec un pas d'ondulation de 2 à 3 mm et une profondeur d'ondulation supérieure de quelques dixièmes de millimètre à la profondeur (typiquement 2/10 mm environ).

Les bagues sont susceptibles d'autres modes de réalisation, par exemple par découpage de languettes repoussées alternativement d'un côté et de l'autre de la bague.

Dans la variante de réalisation montrée en figure 10 (où les éléments correspondants à ceux des figures 1 à 5 sont encore désignés par le même numéro de référence) la partie haute de la tige fémorale 14 est encore découpée et délimite un doigt élastique 24 muni d'une crémaillère 26. La découpe est plus complexe que dans le cas de la figure 1 de façon que sur la partie massive de la tige subsiste un crochet 27 constituant butée limitant l'écartement du doigt 24.

La vis 28 d'appui sur le doigt 24 est desserrée lors de l'introduction de la pièce fémorale jusqu'à appui de l'épaulement 18 sur la corticale. Lorsqu'on commence à serrer la vis 28, le doigt 24 tend à s'écarter par flexion autour d'un point 0 placé dans la partie massive, à proximité de l'extrémité de la découpe. Cette flexion est limitée par la venue en butée du doigt contre le crochet 27. On continue alors à serrer la vis 28 pour obtenir une seconde flexion, qui intervient cette fois autour d'un point différent et tend à appliquer davantage l'épaulement 18, assurant ainsi une seconde précontrainte. L'amplitude des deux mouvements de flexion successifs est limitée à la valeur correspondant aux jeux initiaux j1 et j2 choisis de façon appropriée.

La pièce acétabulaire montrée en figures 11 et 12 présente également une constitution lui permettant de s'adapter aux déformations du squelette et de les absorber. La pièce représentée en figures 11 et 12 est du type à fixation par vissage dans la cavité acétabulaire, le pas de vis pourrait notamment être remplacé par des aspérités assurant un blocage dans les deux dimensions.

La coquille métallique 40 est d'une seule pièce et présente une partie interne non découpée, délimitant un logement légèrement tronconique à épaulement dans lequel s'emboîte à force la cupule 42, généralement en matière plastique, de réception de la boule 22 de la pièce fémorale. Des saignées circonférentielles tronconiques étroites 44 (par exemple de 0,5 mm) et des incisions radiales 46 fractionnent la partie externe de la coquille 40 en une série de doigts élastiques 48 destinés à absorber les déformations osseuses.

Il est possible d'ajuster la raideur des doigts, donc la force nécessaire à leur déformation, par un choix approprié de l'épaisseur de matière qu'on laisse subsister entre la partie interne de la coquille 40 et les doigts. Une épaisseur e d'environ 2 mm donnera souvent des résultats satisfaisant, avec une épaisseur des doigts eux-mêmes d'environ 1,5 mm. Pour augmenter l'élasticité, plusieurs saignées circonférentielles 44 peuvent être disposées en quinconce (au nombre de deux sur la figure 12).

La face externe des doigts présente un filetage auto-taraudant à un pas et avec une forme qui peuvent être classiques, par exemple avec un pas de 9 mm et des dents à 90° sur une face, à 45° sur l'autre. L'une des arêtes, située à l'avant dans le sens d'engagement (indiqué par f sur la figure 11) sera coupante. On adoptera fréquemment un fractionnement par six incisions radiales 46 à 60°.

Dans une variante de réalisation, non représentée, la coquille métallique 40 est constituée en deux parties rapportées l'une sur l'autre. La partie interne rigide constitue logement pour la cupule 42. La partie externe constitue un doublage rapporté, fixé de façon rigide à la partie interne, ayant une constitution similaire à celle montrée sur les figures 11 et 12.

Dans le mode de réalisation montré en figure 13, où les composants correspondant à ceux des figures 11 et 12 sont encore désignés par le même numéro de référence, la coquille métallique 40 présente un logement interne légèrement tronconique destiné à recevoir la cupule 42. Cette cupule présente un ou plusieurs dégagements 50 en forme de croissant, destinés à favoriser la lubrification. La coquille 42 repose sur le fond d'une cavité ménagée dans l'os 52 par des moyens élastiques d'appui formé par une cuvette 54 de profondeur variable, dont le fond s'appuie sur l'os et dont le bord est découpé par des fentes 56 en languettes d'appui sur la partie massive de la coquille. Pour retenir la cuvette sur la coquille, les languettes peuvent comporter une languette repliée 58 qui s'engage dans une gorge ménagée à cet effet au fond d'un évidement de la coquille.

Radialement la coquille s'appuie sur l'os par l'intermédiaire d'une ceinture élastique 60 de forme généralement tronconique, présentant des ondulations de profondeur supérieure à la distance prévue entre la paroi latérale de la partie massive de la coquille et la paroi de la cavité ménagée dans l'os 52.

D'autres modes de réalisation encore sont possibles. Par exemple, la coquille peut comporter deux coques concentriques, la coque interne étant rigide et indéformable tandis que la coque externe est déformable. Un milieu de transmission de pression est alors placé dans une chambre ménagée entre les deux coques.

L'invention ne se limite pas aux modes particuliers de réalisation qui ont été représentés et décrits à titre d'exemple.

**Revendications**

1. Pièce acétabulaire pour prothèse de la hanche, délimitant un logement en forme de calotte sphérique destinée à recevoir une pièce fémorale terminée par une tête en forme de boule, la pièce acétabulaire comprenant une cupule rigide interne (42) en matériau à faible coefficient de frottement contenue dans une coquille (40), caractérisé en ce que la coquille a une zone interne rigide de réception de la cupule (42), seule en contact avec la cupule, et une zone externe, placée à l'extérieur de la zone interne dans le sens radial, présentant une élasticité radiale y compris lorsque la cupule est en place.

2. Pièce acétabulaire selon la revendication 1, caractérisée en que la zone externe est d'une seule pièce avec la zone interne et est fractionnée en plusieurs secteurs constituant des doigts élastiques par des incisions radiales (46) et reliés à la zone interne par une liaison réservée par une saignée circonférentielle (44) ou plusieurs saignées en quinconce.

3. Pièce acétabulaire selon la revendication 2, caractérisée en ce que les saignées circonférentielles sont de forme tronconique.

4. Pièce acétabulaire selon la revendication 1, caractérisée en ce que la zone externe est rapportée rigidement sur la zone interne.

5. Pièce acétabulaire selon la revendication 1, caractérisée en ce que la zone externe est constituée par un revêtement de matériau élastique déformable recouvrant au moins partiellement la zone interne rigide.

6. Pièce acétabulaire selon la revendication 5, caractérisée en ce que le revêtement est constitué par une cuvette élastique et/ou une ceinture élastique portées par le fond et/ou les côtés d'une coque constituant la zone centrale.

7. Pièce acétabulaire selon la revendication 1, caractérisée en ce que la zone interne est constituée par une coque rigide et en ce que la zone externe est constituée par une coque déformable, délimitant avec la coque interne une cavité close occupée par un milieu de répartition de pression.

8. Prothèse de la hanche comportant une pièce acétabulaire et une pièce fémorale comportant une tige (14) de fixation intramédullaire rigide, caractérisée en ce que la pièce acétabulaire est selon l'une quelconque des revendications 1 à 7 et en ce que la tige est revêtue sur une partie de sa longueur d'un matériau déformable, constitué par exemple par une ou plusieurs bagues de rattrapage élastiques.

9. Prothèse de la hanche comportant une pièce acétabulaire et une pièce fémorale comportant une tige (14) de fixation intramédullaire rigide, caractérisée en ce que la pièce acétabulaire est selon l'une quelconque des revendications 1 à 7 et en ce que la tige est découpée à sa partie haute pour constituer un doigt élastique (24) muni d'une crémaillère (26) de retenue dans l'os, et est munie de moyens de pression, tels qu'une vis, permettant d'écarter le doigt (24) de la fibre neutre de la pièce pour ancrer la crémaillère dans la paroi médullaire et mettre la corticale en contrainte axiale.

**Patentansprüche**

1. Gelenkpfannenteil für Hüftprothese, das einen sphärisch-kalottenförmigen Sitz zur Aufnahme eines durch einen kugelförmigen Kopf begrenzten Oberschenkelteils bildet, wobei das Gelenkpfannenteil eine innere starre Pfanne (42) aus einem Material mit geringem Reibungskoeffizienten aufweist, die in einer Lagerschale (40) aufgenommen ist, **dadurch gekennzeichnet**, daß die Lagerschale einen allein in Kontakt mit der Pfanne stehenden starren Innenbereich zur Aufnahme der Pfanne (42) und einen außerhalb des Innenbereichs in Radialrichtung angeordneten Außenbereich hat, der eine radiale Elastizität aufweist, auch wenn sich die Pfanne an ihrem Platz befindet.

2. Gelenkpfannenteil nach Anspruch 1, dadurch gekennzeichnet, daß der Außenbereich einstückig mit dem Innenbereich ausgebildet und in mehrere Sektoren aufgeteilt ist, die durch radiale Einschnitte (46) klassische Finger bilden und am Innenbereich durch eine Verbindung miteinander verbunden sind, die durch einen Umfangseinschnitt (44) oder mehrere gegeneinander versetzte Einschnitte bestimmt wird.

3. Gelenkpfannenteil durch Anspruch 2, dadurch gekennzeichnet, daß die Umfangseinschnitte kegelstumpfförmig sind.

4. Gelenkpfannenteil nach Anspruch 1, dadurch gekennzeichnet, daß der Außenbereich fest auf den Innenbereich angeordnet ist.

5. Gelenkpfannenteil nach Anspruch 1, dadurch gekennzeichnet, daß der Außenbereich durch einen Überzug aus elastisch verformbarem Material gebildet ist, der wenigstens teilweise den starren Innenbereich abdeckt.

6. Gelenkpfannenteil nach Anspruch 5, dadurch gekennzeichnet, daß der Überzug durch eine elastische Schale und/oder einen elastischen Ring gebildet ist, die durch den Boden und/oder die Seiten einer den Mitteilteil bildenden Schale getragen werden.

7. Gelenkpfannenteil nach Anspruch 1, dadurch gekennzeichnet, daß der Innenbereich durch eine starre Schale und der Außenbereich durch eine verformbare Schale gebildet ist, die mit der inneren Schale einen geschlossenen Hohlraum bildet, der mit einem Druckverteilungsmillieu gefüllt ist.

8. Hüftprothese mit einem Gelenkpfannenteil und einem Oberschenkelteil, das einen starren intramedullären Befestigungsschaft aufweist, dadurch gekennzeichnet, daß das Gelenkpfannenteil gemäß einem der Ansprüche 1 bis 7 ausgebildet ist und daß der Schaft auf einem Teil seiner Länge mit einem verformbaren Material umhüllt ist, das beispielsweise durch einen oder mehrere elastische Anpaßringe gebildet ist.

9. Hüftprothese mit einem Gelenkpfannenteil und einem Oberschenkelteil, das einen starren intramedullären Befestigungsschaft aufsweist, dadurch gekennzeichnet, daß das Gelenkpfannenteil gemäß einem der Ansprüche 1 bis 7 ausgebildet und daß der Schaft an seinem Oberteil eingeschnitten ist zur Bildung eines mit einer Verzahnung (26) für die Halterung im Knochen versehenen elastischen Fingers (24) und Druckmittel aufweist, wie z.B. eine Schraube, die es ermöglichen, den Finger (24) von der neutralen Phase des Teils wegzubewegen, um die Verzahnung in der Medullärwand zu verankern und um die Rinde in axiale Spannung zu versetzen.

## Claims

1. Acetabulum unit for hip joint prosthesis, defining a cavity having a spherical cap shape, for receiving a femoral unit having a ball shaped end head, comprising an internal rigid cup (42) of a material having a low friction coefficient contained in a body (40), characterized in that the body has an inner rigid zone for receiving the cup (42) alone in contact with the cup, and an outer zone, placed out of the inner zone in the radial direction, having a radial elasticity, even when the cup is in place.

2. Acetabulum unit according to claim 1, characterized in that the outer zone is integral with the inner zone and is fractionated into a plurality of sectors which constitute resilient fingers by radial slots (46) and are joined to the inner zone by a connection reserved by a circumferential groove (44) or a plurality of grooves in a quincunx arrangement.

3. Acetabulum unit according to claim 2, characterized in that the circumferential grooves are frusto-conical in shape.

4. Acetabulum unit according to claim 1, characterized in that the external zone is rigidly connected to the inner zone.

5. Acetabulum unit according to claim 1, characterized in that the outer zone consists of a liner of resiliently deformable material at least partially covering the rigid inner zone.

6. Acetabulum unit according to claim 5, characterized in that the lining consists of a resilient cup and/or a resilient belt carried by the bottom wall and/or the sides of a shell constituting the central zone.

7. Acetabulum unit according to claim 1, characterized in that the inner zone consists of a rigid shell and in that the outer zone consists of a deformable shell which cooperates with the inner shell to define a closed chamber accommodating a pressure distribution medium.

8. Hip prosthesis comprising an acetabulum unit and a femoral unit having a rigid intramedular connection stem (14), characterized in that the acetabulum unit is according to any one of claims 1-7 and in that part of the length of the stem is covered, with a deformable material, constituted for instance by one or a plurality of resilient compensation rings.

9. Hip prosthesis comprising an acetabulum unit and a femoral unit having a rigid intramedular connection stem (14), characterized in that the acetabulum unit is according to any one of claims 1-7 and in that the stem is cut out in an upper portion thereof for constituting a resilient finger (24) formed with a rack (26) for anchoring in the bone and is provided with pressure means, such as a screw, for forcing the finger (24) apart from the neutral fiber of the unit for anchoring the rack into the medular wall and to impress an axial stress on the cortical.

FIG.1.

FIG.2.

FIG.3.

FIG.4.

FIG.5.

FIG.7.

FIG.6.

22

18

FIG.8

36

14

34

34

FIG.9.

38

32

32

FIG.9A.

38

FIG.11.

40

XII

46

48

46

FIG.12.

40    42

44

e

FIG.10.

28

26
27    j2    j1

24

18

14

O

FIG.13.

58    52    56    54    40

60    42

50